# EUROPÄISCHE PATENTSCHRIFT

(11) **EP 3 909 632 B1**
(45) Veröffentlichungstag und Bekanntmachung des Hinweises auf die Patenterteilung: **22.03.2023**
(21) Anmeldenummer: 21020212.3
(22) Anmeldetag: 19.04.2021
(51) Int. Cl.: A61M 16/01, A61M 16/00, A61M 16/10, A61M 16/08, A61M 16/12, A61M 16/20

(54) **BEATMUNGSGERÄT ZUR BEATMUNG EINES LEBEWESENS**
VENTILATOR FOR VENTILATING A LIVING ORGANISM
APPAREIL RESPIRATOIRE DESTINÉ À LA RESPIRATION D'UN ÊTRE VIVANT

(30) Priorität: 23.04.2020 DE 102020002439
(43) Veröffentlichungstag der Anmeldung: 17.11.2021
(73) Patentinhaber: Löwenstein Medical Technology S.A., 2557 Luxembourg (LU)
(72) Erfinder: BORM, Petrus Johannes Josephus, 5627 HP Eidhoven (NL)
(74) Vertreter: Marx, Thomas

(56) Entgegenhaltungen:
- EP-A2- 0 282 675
- WO-A1-97/19719
- GB-A- 1 206 307
- GB-A- 1 358 473
- US-A- 781 939
- US-A1- 2006 249 153

## Beschreibung

Die Erfindung betrifft ein Beatmungsgerät zur Beatmung eines Lebewesens.

Beatmungsgeräte werden unter anderem zur Beatmung von Lebewesen mit einem Atemgas eingesetzt. Die Beatmung von Patienten in Krankenhäusern oder im häuslichen Umfeld mit einem Atemgas ist ein übliches Anwendungsfeld solcher Beatmungsgeräte. Im Bereich der Anästhesie können Beatmungsgeräte zum Teil auch als Anästhesiegeräte verwendet werden. Dazu wird dem Atemgas, welches dem Patienten zugeführt wird, ein zusätzliches Narkosegas zugemischt.

Narkosegase, zum Beispiel Xenon, sind sehr teuer. Daher ist es vorteilhaft, wenn der Anteil des Narkosegases, welches nach dem Ausatmen noch in dem Atemgas vorhanden ist herausgefiltert oder wiederverwendet werden kann. Letzteres kann durch ein Beatmungsgerät realisiert werden, bei dem das Atemgas in einem Kreislauf geführt wird. D.h. es ist nötig aus dem ausgeatmeten Gasgemisch zum einen das ausgeatmete CO₂ herauszufiltern und zum anderen verbrauchten Sauerstoff sowie Narkosegas wieder zuzusetzen.

In der Regel findet bei solchen Geräten eine kontinuierliche Zufuhr von Narkosegas statt. Für eine weitere Einsparung von Narkosegas ist es möglich, das Narkosegas nur in kleinen Mengen in Form von kurzen Pulsen dem Atemgas beizumischen. Zusätzlich bietet das pulsförmige Einleiten des Narkosegases, z.B. über sehr schnelle Ventile, eine günstige und auch genaue Form der Dosierung.

Solche Beatmungsgeräte sind bekannt. Das Dokument US 2006/249153 A1 offenbart ein Beatmungsgerät mit einem Ventil, welches einen pulsierenden Gasfluss dämpft. In dieses Ventil ist eine Feder eingebaut, welche dazu genutzt wird um einen Druck einzustellen, welcher benötigt wird, um den Gasfluss aufrecht zu erhalten.

Ein Nachteil dieser pulsförmigen Zugabe von Narkosegas besteht darin, dass der dadurch entstehende Impuls auf den gesamten Gasfluss übertragen wird. Dadurch wird unter anderem die Messung des Gasflusses durch plötzlich auftretende Druckspitzen empfindlich gestört. Diese Impulse können mitunter eine solche Stärke haben, dass diese sich bis zum Patienteninterface fortsetzen und auch vom Patienten oder dem behandelnden Personal als störend wahrgenommen werden können.

Die Aufgabe der vorliegenden Erfindung ist es, ein Beatmungsgerät bereit zu stellen, welches eine pulsförmige Zugabe von Narkosegas zum Atemgas zulässt, den dadurch gestörten Gasfluss aber beruhigen bzw. in einen stetigen Fluss überführen kann.

Gemäß einem Aspekt der Erfindung wir die Aufgabe mit einem Beatmungsgerät mit einer Gasdosierungseinheit und einem Pulsdämpfer nach Anspruch 1 gelöst. Das Beatmungsgerät umfasst einen Druckerzeuger, eine Gasdosierungseinheit, eine pneumatische Hauptleitung, durch welche das Atemgas zum Patienteninterface geleitet wird, sowie einen Pulsdämpfer.

Die Gasdosierungseinheit ist so eingerichtet Atemgas oder Zusätze zum Atemgas wie zum Beispiel Außenluft und/oder Sauerstoff und/oder Narkosegas in die pneumatische Hauptleitung zu leiten. Die Einleitung von Atemgas oder Zusätzen zum Atemgas ist auf bestimmte Mengen oder Volumina ausgelegt. Der Druckerzeuger ist dazu eingerichtet, das Atemgas in die pneumatische Hauptleitung zum Patienteninterface zu fördern.

In einer Ausführungsform der Erfindung ist die Dosierungseinheit eingerichtet, dass das Atemgas oder die Zusätze zum Atemgas bereitzustellen indem Luft (z.B. Außenluft oder aus einer Druckluftflasche) und/oder Sauerstoff und/oder Narkosegas gemischt werden. Zudem verfügt die Gasdosierungseinheit über zumindest ein Ventil. Die genannten Impulswellen können beispielsweise durch die gepulste, schnelle Zuführung von bestimmten Mengen oder Volumina an Gas oder Gasgemischen durch die Dosierungseinheit entstehen.

Der Pulsdämpfer des Beatmungsgeräts ist beispielsweise so ausgelegt und eingerichtet, dass Impulswellen der Atemgasquelle und/oder der Gasdosierungseinheit durch ebenjenen Pulsdämpfer gedämpft werden.

In einer weiteren Ausführung des Beatmungsgeräts ist der Pulsdämpfer in Durchflussrichtung - ausgehend von der Dosierungseinheit und dem Druckgeber zum Patienteninterface - nach der Dosierungseinheit aber vor der pneumatischen Hauptleitung angeordnet. Die Gasdosierungseinheit ist dabei beispielsweise in Durchflussrichtung vor dem Druckerzeuger angeordnet, wobei der Druckerzeuger wiederum in Durchflussrichtung vor dem Patienteninterface angeordnet ist. Somit wäre zum Beispiel die Dämpfung der Impulswellen, welche durch die Dosierungseinheit erzeugt werden können, gegeben. Die Durchflussrichtung wird dabei beispielsweise durch eine nicht näher beschriebene Umwälzeinheit, beispielsweise eine Druckpumpe, bestimmt.

Auch eine Anordnung von beispielsweise mehreren Pulsdämpfern zwischen Gasdosierungseinheit und pneumatischer Hauptleitung ist möglich.

In einer weiteren Ausführung der Erfindung verfügt der Pulsdämpfer über einen Anschluss für die Gasdosierungseinheit sowie einen Anschluss für die pneumatische Hauptleitung. Diese Anschlüsse sind so zu verstehen, dass auch eine pneumatische Anordnung des Pulsdämpfers beispielsweise zwischen dem Druckerzeuger und der pneumatischen Hauptleitung realisiert werden kann

Der Pulsdämpfer ist als elastomeres Bauteil ausgebildet. Der Pulsdämpfer umfasst dabei einen schlauchartigen Zulauf, welcher sich zu einer Kammer aufweitet. Die Kammer wiederum geht allmählich in einen schlauchartigen Auslauf über. Das Bauteil ist beispielsweise so ausgebildet, dass die die Aufweitung vom Zulauf zu der Kammer in einem sehr viel größeren Winkel erfolgt als der allmähliche Übergang der Kammer zu dem Auslauf.

In einer weiteren Ausführungsform der Erfindung ist der Pulsdämpfer so eingerichtet, dass sich die das Volumen u₁ des pulsartig durch die Dosierungseinheit zugeführten Gases in der Kammer ausbreiten kann und in einen weniger pulsartigen oder stetigen Fluss übergeht. Der zu erreichende, weniger pulsartige bzw. stetige Fluss in der pneumatischen Hauptleitung wird beispielsweise durch eine nicht näher beschriebene Umwälzeinheit vorgegeben.

In einer weiteren Ausführungsform der Erfindung beträgt der zu erreichende, stetige Fluss in der pneumatischen Hauptleitung 20 bis 100 L/min, bevorzugt 30 bis 80 L/min.

In einer weiteren Ausführungsform der Erfindung beträgt das Volumen u₁ des pulsartig durch die Dosierungseinheit zugeführte Gas 1 bis 25 mL, bevorzugt 3 bis 15 mL.

In einer Ausführungsform ist der Pulsdämpfer aus Silikon gefertigt. In einer weiteren Ausführungsform weißt der Pulsdämpfer eine gewisse Rauigkeit auf.

In einer weiteren Ausführungsform der Erfindung umfasst die Kammer des Pulsdämpfers eine Oberfläche, welche beispielsweise zumindest teilweise mit einem Dämmmaterial ausgekleidet ist.

Der Pulsdämpfer ist in einem Pulsdämpfergehäuse platziert, welches den Pulsdämpfer zum Beispiel mindestens teilweise bzw. abschnittsweise haltert. Das Pulsdämpfergehäuse weist mindestens ein Federelement auf. Das Federelement ist beispielsweise so ausgebildet und eingerichtet, dass eine auf den Fluss in der pneumatischen Hauptleitung abgestimmte Dämpferstärke erreicht werden kann.

In manchen Ausführungsformen beträgt die Federkonstante eines der Federelemente zwischen 25 N/mm und 150 N/mm, bevorzugt zwischen 45 N/mm und 66 N/mm.

In einer weiteren Ausführungsform sind der Pulsdämpfer, das Pulsdämpfergehäuse sowie die Federelemente so eingerichtet und ausgebildet, dass sie auf die eingeleitete Menge oder das eingeleitete Volumen u₁ des Gases aus der Gasdosierungseinheit und/oder auf den Durchfluss in der pneumatischen Hauptleitung abgestimmt sind.

In weiteren Ausführungsformen ist die Dämpferstärke des Pulsdämpfers durch Versetzen des zumindest einen Federelements einstellbar.

In manchen Ausführungsformen ist daran gedacht, dass die Gehäuseteile über Scharnier miteinander verbunden sind, so dass sich ein Gehäuseteil beim Expandieren des Pulsdämpfers an einem Ende stärker anhebt als an dem gegenüberliegenden Ende.

Auch ist in manchen Ausführungsformen daran gedacht, dass die Dämpferstärke des Pulsdämpfers über die Federstärke bzw. die Federkonstante des zumindest einen Federelements einstellbar ist

Das Funktionsprinzip des Pulsdämpfers basiert beispielsweise auf der Expansion mit nachfolgender Kontraktion des beispielhaft elastomeren Bauteils bei pulsartiger Einleitung des Gases durch die Gasdosierungseinheit. Wird eine bestimmte Menge Gas durch die Gasdosierungseinheit pulsartig in die pneumatische Hauptleitung eingeleitet, so erhöht sich der Druck bzw. der Fluss in der pneumatischen Hauptleitung sprunghaft. Wird der Pulsdämpfer, welcher auch einen gewissen pneumatischen Widerstand darstellt, zwischen Gasdosierungseinheit und der pneumatischen Hauptleitung eingesetzt, baut sich durch die pulsartige Einleitung des Gases ein Druck vor dem Pulsdämpfer auf. Diesem Druckanstieg kann der Pulsdämpfer nachgeben und dabei expandieren und so durch Volumenzunahme den Druckanstieg ausgleichen. Dabei dehnt sich die zugeleitete Gasmenge innerhalb des Pulsdämpfers aus. Zieht sich der Pulsdämpfer wieder zusammen, so wird das zugenommene Volumen bzw. die darin enthaltene Gasmenge allmählich dem Fluss in der pneumatischen Hauptleitung zugeführt. Dadurch wird die initiale Druck- bzw. Flusserhöhung durch die Gaseinleitung nicht auf die pneumatische Hauptleitung übertragen. Die beispielhaft an einem möglichen Pulsdämpfergehäuse angebrachten Federelemente können dazu dienen, die Dämpfstärke einzustellen und auch die Kontraktion des Pulsdämpfers zu unterstützen.

### Figuren

- Figur 1:: Schematische Darstellung einer beispielhaften Ausführungsform eines Beatmungsgeräts
- Figur 2:: Schematische Darstellung einer beispielhaften Ausführungsform eines Pulsdämpfers in perspektivischer Sicht (a), in Aufsicht (b) und Querschnitt in Seitansicht
- Figur 3:: Schematische Darstellung einer beispielhaften Ausführungsform eines Pulsdämpfers mit Pulsdämpfergehäuse in perspektivischer Ansicht
- Figur 4:: Schematische Darstellung einer beispielhaften Ausführungsform eines Pulsdämpfers mit Pulsdämpfergehäuse in perspektivischer Ansicht
- Figur 5:: Schematische Darstellung einer beispielhaften Ausführungsform eines Pulsdämpfers mit Pulsdämpfergehäuse in perspektivischer Ansicht

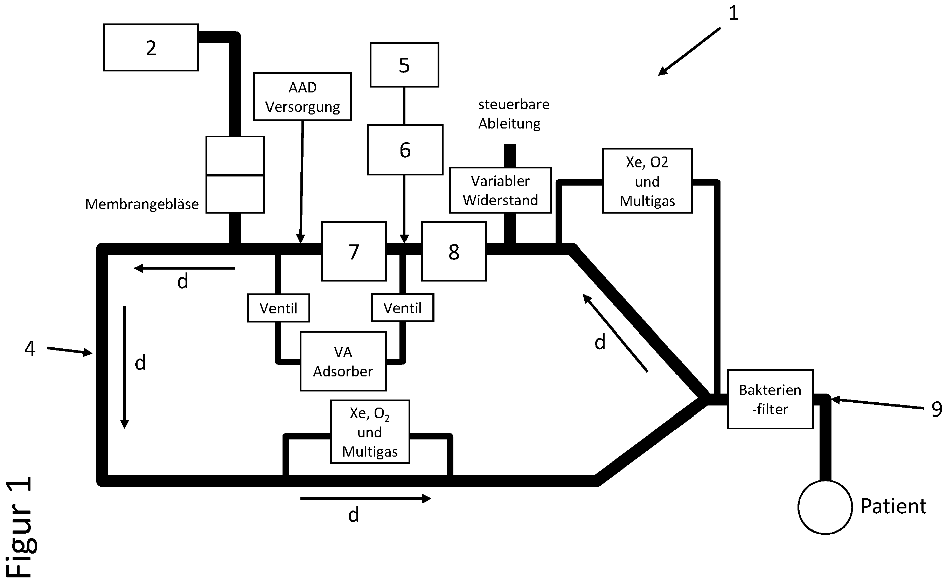

Figur 1 zeigt eine schematische Darstellung einer beispielhaften Ausführungsform eines Beatmungsgeräts (1), welches beispielhaft über einen geschlossenen Gaskreislauf mit einem CO₂-Absorber (8) verfügt. Das in Figur 1 dargestellte Beatmungsgerät (1) umfasst einen Druckerzeuger (2), eine Gasdosierungseinheit (5), eine pneumatische Hauptleitung (4) sowie einen Übergang zu dem Patienteninterface (9).

Über die Gasdosierungseinheit (5) wird bei Bedarf Narkosegas und/oder Sauerstoff und/oder Luft pulsartig in die pneumatische Hauptleitung eingeleitet. Der Druckerzeuger (2) fördert das Atemgas über die pneumatische Hauptleitung (4) zu dem Patienteninterface (9). Um die Impulswellen der pulsartigen Einleitung von Narkosegas und/oder Sauerstoff in die Hauptleitung (4) zu dämpfen ist der Pulsdämpfer (6) in Figur 1 beispielhaft in Gasflussrichtung (d) nach der Gasdosierungseinheit (5) und vor der pneumatischen Hauptleitung (4) angeordnet. Der Pulsdämpfer (6) ist so eingerichtet, dass Impulswellen, entstehend durch die pulsartige Einleitung von Gas aus der Gasdosierungseinheit (5), gedämpft werden. Eine Umwälzeinheit (7) bestimmt den Durchfluss in der pneumatischen Hauptleitung und bewirkt eine schnelle Durchmischung des im Kreislauf geführten Atemgases mit den zugesetzten Gasen wie z.B. Sauerstoff oder Narkosegas. Der CO₂-Absorber (8) entfernt das ausgeatmete CO₂ des Patienten aus dem Atemgas in der pneumatischen Hauptleitung (4).

Neben der in Figur 1 gezeigten und beschriebenen Anordnung des Pulsdämpfers (6) ergeben sich auch weitere mögliche Anordnungen.

Der Pulsdämpfer (6) kann beispielsweise auch in einer Weise entlang der pneumatischen Hauptleitung (4) angeordnet sein, dass der Pulsdämpfer (6) in einer Nebenleitung oder als eine Art Sackgasse von der Hauptleitung (4) abzweigt.

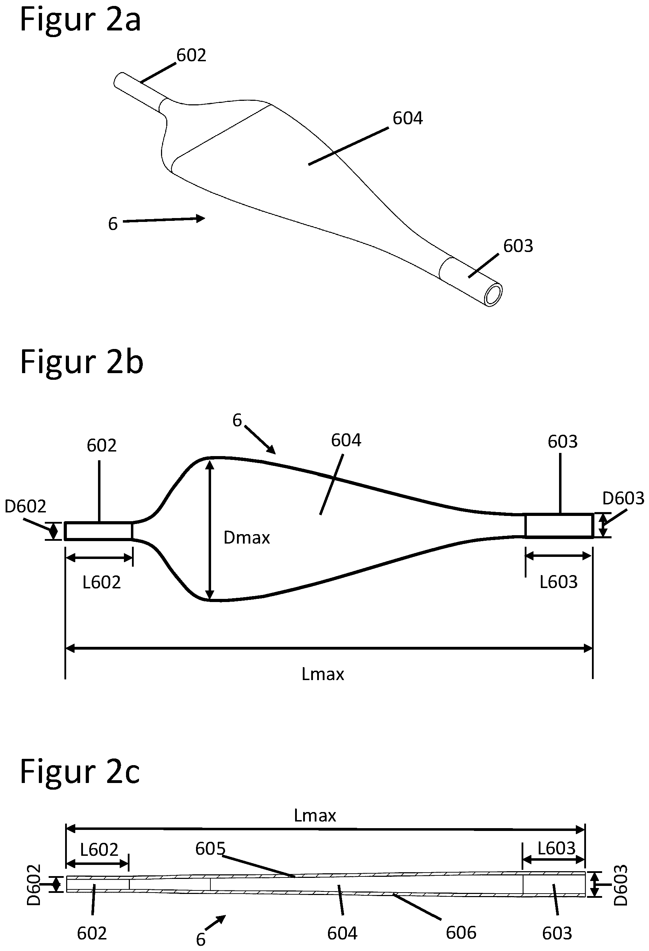

Figuren 2a, b, c zeigen ein schematische Darstellungen einer beispielhaften Ausführung des Pulsdämpfers (6). Figur 2a zeigt dabei eine perspektivische Ansicht, 2b eine Aufsicht und 2c einen schematischen Querschnitt in der Seitenansicht. Der als schlauchartige Zulauf ausgebildete Anschluss (602), welcher beispielhaft die Gasdosierungseinheit (5) mit dem Pulsdämpfer (6) verbindet, weitet sich zu einer Kammer (604) auf. Die Kammer (604) ist so eingerichtet und ausgebildet, dass sich das von der Gasdosiereinheit (5) eingespeiste Gas mit einem Volumen u₁ ausbreiten kann und in einen weniger pulsartigen oder stetigen Fluss übergehen kann. Dazu ist beispielsweise der Pulsdämpfer (6) als Ganzes oder zumindest die Kammer als elastomeres Bauteil ausgebildet, sodass sich die Kammer erweitern und auch wieder zusammenziehen kann. Die Kammer (604) geht allmählich zulaufend in einen als schlauchartigen Zulauf ausgebildeten Anschluss (603) über, welcher den Pulsdämpfer mit zum Beispiel der pneumatischen Hauptleitung (4) verbindet. Insgesamt ist der Pulsdämpfer als hohl und offen anzusehen, sodass der Gasfluss in den Pulsdämpfer (6) hinein, von Anschluss (602) zu Anschluss (603) innerhalb des Pulsdämpfers (6) und dann aus den Pulsdämpfer (6) hinaus möglich ist. Im nicht-erweiterten Zustand des Pulsdämpfers (6) sind die Kammeroberseite (605) und die Kammerunterseite (606) des Pulsdämpfers (6) beabstandet voneinander, es ist aber auch möglich, dass sich zum Beispiel gegenüberliegende Wände, also beispielsweise die Kammeroberseite (605) und die Kammerunterseite (606) berühren können.

Wie in Figur 2c zu erkennen, ist der Pulsdämpfer (6) beispielsweise flach aufgebaut, die Höhe im nicht-erweiterten Zustand ist also kleiner als die maximale Breite. Beispielsweise liegt die Höhe des Pulsdämpfers im Bereich der Durchmesser der Anschlüsse (602, 603). In weiteren Ausführungsformen kann der Pulsdämpfer aber auch einen runden Querschnitt entlang der Durchflussrichtung aufweisen, das heißt unter anderem, dass die höchste Stelle im Bereich der breitesten Stelle angeordnet ist und auch die maximale Höhe der maximalen Breite entspricht bzw. der Durchmesser an dieser Stelle der maximalen Breite entspricht.

Die Wandstärke des Pulsdämpfers ist zwischen 0,5 mm und 5 mm, bevorzugt zwischen 0,7 mm und 2,5 mm.

Die Gesamtlänge Lmax des Pulsdämpfers ist zwischen 30 mm und 500 mm, bevorzugt zwischen 60 mm und 250 mm.

Die maximale Breite Dmax des Pulsdämpfers ist zwischen 20 mm und 100 mm, bevorzugt zwischen 30 mm und 70 mm.

Das Verhältnis der jeweiligen Anschlusslänge der Anschlüsse (L602, L603) zu der Gesamtlänge Lmax des Pulsdämpfers (6) beträgt beispielsweise von 1:3 bis 1:15, bevorzugt von 1:5 bis 1:10.

Das Verhältnis der Breite der Anschlüsse (D602, D603) zu der maximalen Breite Dmax des Pulsdämpfers beträgt beispielsweise von 1:5 bis 1:25, bevorzugt von 1:7 bis 1:17. Sind die Anschlüsse beispielweise zylinderförmig ausgebildet, so wäre die Breite der Anschlüsse gleichbedeutend mit dem Durchmesser. In der Figur 2 gezeigten beispielhaften Ausführungsform ist die Breite D602 des Anschlusses (602) geringfügig kleiner als die Breite D603 des Anschlusses (603). Für weitere, nicht gezeigte, beispielhafte Ausführungsformen können die Breiten D602 und D603 auch gleich sein. In weiteren Ausführungsformen können die Breiten D602, D603 der Anschlüsse (602, 603) beispielsweise auf die Anschlüsse der vorhergehenden und nachfolgenden Leitungen, zum Beispiel den Anschluss der Gasdosierungseinheit (5) oder der pneumatischen Hauptleitung (4), angepasst sein.

Das Verhältnis der maximalen Breite Dmax zu der Gesamtlänge Lmax des Pulsdämpfers (6) beträgt beispielsweise von 1:1 bis 1:10, bevorzugt von 1:2 bis 1:6.

Die breiteste Stelle des Pulsdämpfers (6) mit einer Breite Dmax ist befindet sich zwischen 0,07 und 0,5 der Gesamtlänge Lmax in Durchflussrichtung, also von Anschluss (602) in Richtung Anschluss (603). Bevorzugt befindet sich die breiteste Stelle zwischen 0,16 und 0,4 der Gesamtlänge Lmax.

Es ist zu verstehen, dass die in Figur 2 schematisch dargestellte Form des Pulsdämpfers (6) eine beispielhafte Form darstellt und der Pulsdämpfer (6) auch beliebige andere Formen annehmen kann.

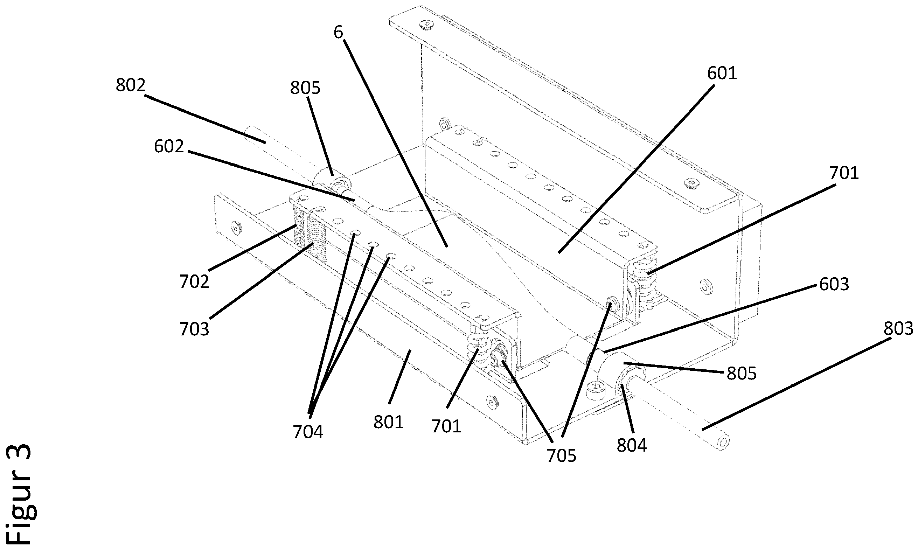

Figuren 3 bis 5 zeigen perspektivische, schematische Darstellungen einer beispielhaften Ausführung des Pulsdämpfers (6) in einer beispielhaften Ausführung des Pulsdämpfergehäuses, welches ein oberes Gehäuseteil (601) und ein unteres Gehäuseteil (801) umfasst.

Das obere Gehäuseteil (601) ist beispielsweise so ausgebildet und eingerichtet, dass es höher liegende Ränder und einen tieferliegenden Mittelteil aufweist. Der tieferliegende Mittelteil ist unmittelbar auf dem Pulsdämpfer (6) gelegen und deckt beispielsweise die Kammer (604) des Pulsdämpfers (6) vollständig ab. Der tieferliegende Mittelteil des oberen Gehäuseteils ist beispielhaft flach dargestellt. In weiteren beispielhaften Ausführungen kann dieser tieferliegende Mittelteil auch die geometrische Form der Kammer (604) des Pulsdämpfers (6) in nicht-expandiertem Zustand zumindest teilweise und/oder grob abbilden. Die höher liegenden Ränder des oberen Gehäuseteils (601) dienen dazu, dass die Gehäuseteile (601, 801) durch Federelemente (701, 702, 703) verbunden werden können. Dazu sind entsprechende Löcher (704) in den Gehäuseteilen (601, 801) vorhanden, in welche die Federelemente (701, 702, 703) beispielsweise eingehängt und/oder auf andere Weise reversibel und/oder permanent befestigt werden können. Zusätzlich sind die Gehäuseteile (601, 801) über eine Art Scharnier (705) im vorderen Teil - das heißt auf der Seite des Anschlusses (603) - verbunden. Das Scharnier (705) bewirkt in der beispielhaften Ausführungsform, dass sich das obere Gehäuseteil (601) beim Expandieren des Pulsdämpfers (6) am hinteren Ende - also auf der Seite des Anschlusses (602) - anheben kann. Es erlaubt also eine gewisse Rotation des oberen Gehäuseteils (601) um die Scharnierachse. Beispielsweise kann das Scharnier (705) - auch in Kombination mit einem Federelement, z.B. Federelement (701) - auch so eingerichtet sein, dass auch ein Anheben des Gehäuseteils (601) im Bereich des Anschlusses (603) bzw. Scharniers (705) möglich ist. Das Gehäuseteile (601) kann sich an einem Ende also stärker anheben als an dem gegenüberliegenden Ende. Dadurch kann gewährleistet sein, dass auch bei größeren Drücken im Pulsdämpfer (6) ein beruhigter Fluss entstehen kann. Dazu ist beispielweise die Federkonstante des Federelements (701) im Bereich des Anschlusses (603) größer als der übrigen Federelemente.

Die angebrachten Federelemente (701, 702, 703) wiederum dienen zum einen als Widerstand gegen das Anheben im hinteren Teil des oberen Gehäuseteils (601), zum anderen dienen die Federelemente (701, 702, 703) auch der Unterstützung zum Zusammenziehen und damit verbundenen Auslassen des Gases aus dem Pulsdämpfer (6) in beispielsweise die pneumatische Hauptleitung (4).

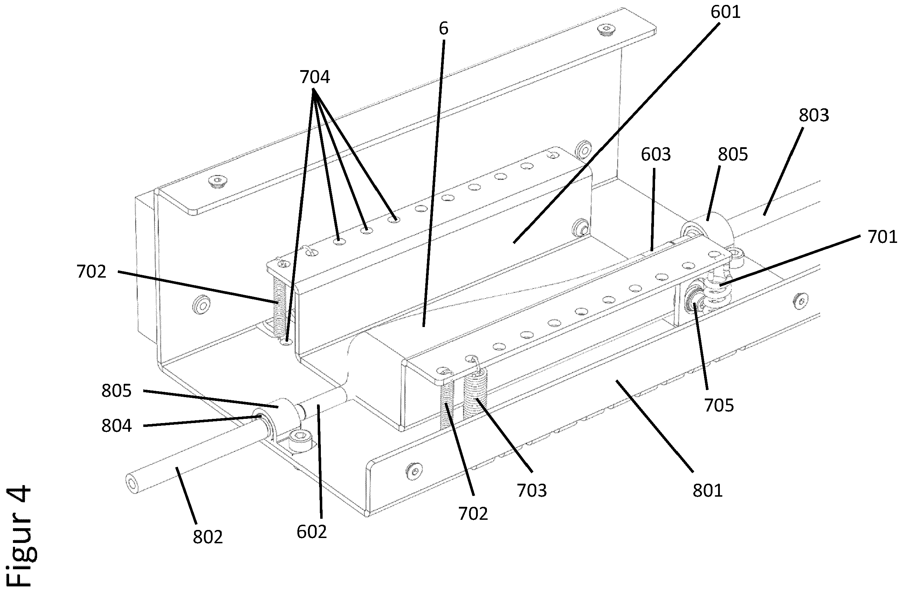

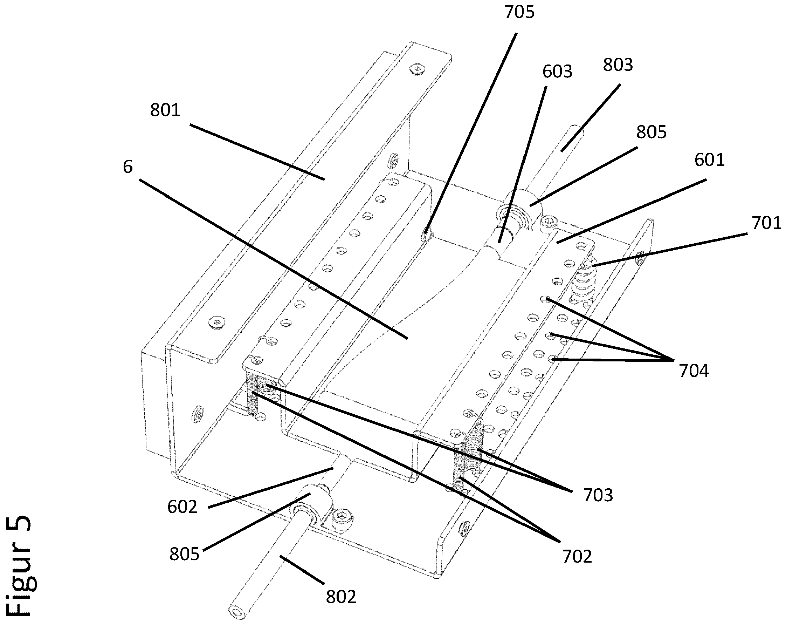

Die eingebrachten Löcher (704) in den Gehäuseteilen (601, 801) dienen neben der Halterung der Federelemente (701, 702, 703) auch dazu, die Dämpferstärke - also den Widerstand gegen die Expansion des Pulsdämpfers und die Unterstützung des Zusammenziehens - nach Bedarf einzustellen. Werden die Federelemente (702) und (703) beispielsweise in weiter vorne liegende Löcher eingehängt, so ergibt sich ein schwächerer Widerstand gegen die Expansion und somit auch eine schwächere Unterstützung des Zusammenziehens des Pulsdämpfers (6). Neben der hier beispielhaft gezeigten Ausführungsform mit insgesamt 6 Federelementen (je Seite drei Federelemente (701, 702, 703), insbesondere in Figuren 4 und 5 erkennbar) sind in weiteren Ausführungsformen auch der Einsatz von mehr oder weniger Federelementen und auch die Kombination verschiedener Federstärken möglich. So kann zum Beispiel in einer weiteren, nicht gezeigten Ausführungsform das obere Gehäuseteil (601) mit dem unteren Gehäuseteil (801) nur über ein Federelement (701) je Seite - also insgesamt zwei Federelemente (701) - verbunden, welches wiederum an der in Figuren 3 bis 6 gezeigte Position des Federelements (702) platziert ist. Weiter kann in weiteren beispielhaften Ausführungsformen die Anzahl und/oder der Abstand zwischen den Löchern (704) für die Federelemente variieren. Insbesondere in Figur 5 ist zu erkennen, dass das untere Gehäuseteil (801) Löcher (704) für die Fixierung der Federelemente (701, 702, 703) aufweist.

Ein Versetzen der Federelemente (701, 702, 703) führt zu einer Veränderung der Dämpferstärke, welche so einstellbar ist. Auch ist ein Einstellen der Dämpferstärke über die Federstärke bzw. die Federkonstante der verwendeten Federelemente (701, 702, 703) möglich.

In den Figuren 3 bis 5 werden jeweils drei Federelemente pro Seite, also insgesamt sechs Federelemente, gezeigt. In weiteren Ausführungen können auch mehr oder weniger Federelemente angeordnet sein. Auch die Position der Federelemente und/oder der Befestigungspunkte, wie z.B. die Löcher (704) können variieren.

Das untere Gehäuseteil (801) umfasst bespielhaft zwei Rohhalterungen (805). Wie in Figuren 3 bis 5 schematisch dargestellt, sind diese Rohrhalterungen so ausgebildet, dass das Verbindungsstück (804), welches die Anschlüsse (602, 603) des Pulsdämpfers (6) mit den Anschlüssen (802, 803) der weiteren Leitungen des Beatmungsgeräts (1) verbindet - beispielsweise über Anschluss (802) mit der Gasdosierungseinheit (5). Das Verbindungsstück (804) ist dabei jeweils so ausgebildet, dass es auf die Abmessungen der jeweiligen Anschlüsse (602, 603, 802, 803) abgestimmt ist. Je nach geometrischer Form zum Beispiel des Verbindungsstücks (804) kann auch die Rohrhalterung (805) auf dieses Verbindungsstück angepasst sein, sodass beide über die gleiche oder zumindest ähnliche geometrische Form verfügen.

Die Gehäuseteile (601, 801) sind beispielsweise aus Aluminium hergestellt. Aber auch andere stabile Materialien wie Kunststoffe oder Stahl sind möglich.

### Bezugszeichenliste:

- 1: Beatmungsgerät
- 2: Druckerzeuger
- 4: pneumatische Hauptleitung
- 5: Gasdosierungseinheit
- 6: Pulsdämpfer
- 7: Umwälzeinheit
- 8: CO₂-Absorber
- 9: Patienteninterface
- 601: (oberes) Gehäuseteil
- 602: Anschluss
- 603: Anschluss
- 604: Kammer
- 605: Kammeroberseite
- 606: Kammerunterseite
- 701: Federelement
- 702: Federelement
- 703: Federelement
- 704: Loch
- 705: Scharnier
- 801: (unteres) Gehäuseteil
- 802: Anschluss
- 803: Anschluss
- 804: Verbindungsstück
- 805: Rohrhalterung
- d: Durchflussrichtung

## Patentansprüche

1. Beatmungsgerät (1) mit einem Druckerzeuger (2), einer Gasdosierungseinheit (5) und einer pneumatischen Hauptleitung (4), die Atemgas zu einem Patienteninterface leitet, wobei
- die Gasdosierungseinheit (5) eingerichtet ist als Atemgas Außenluft und/oder Sauerstoff und/oder Narkosegas in die pneumatische Hauptleitung (4) zu leiten, und
- der Druckerzeuger (2) eingerichtet ist, Atemgas in die pneumatische Hauptleitung zu dem Patienteninterface (9) zu fördern,
wobei die Gasdosierungseinheit (5) eingerichtet ist, bestimmte Mengen oder Volumina an Atemgas in die pneumatische Hauptleitung (4) zu liefern, wobei das Beatmungsgerät einen Pulsdämpfer (6) aufweist, wobei der Pulsdämpfer (6) in einem Pulsdämpfergehäuse, umfassend einen oberen Gehäuseteil (601) und einen unteren Gehäuseteil (801), platziert ist, welches den Pulsdämpfer (6) zumindest teilweise oder abschnittsweise haltert, wobei das Pulsdämpfergehäuse (601) zumindest ein Federelement (701, 702, 703) aufweist, **gekennzeichnet dadurch, dass** der Pulsdämpfer (6) als elastomeres Bauteil ausgebildet ist, mit einem schlauchartigen Zulauf von einem ersten Anschluss (602) der sich zu einer Kammer (604) aufweitet, wobei die Kammer (604) allmählich in einen schlauchartigen Auslauf zu einem zweiten Anschluss (603) übergeht.

2. Beatmungsgerät (1) nach Anspruch 1, wobei die Gasdosierungseinheit (5) eingerichtet ist, Atemgas bereitzustellen indem Luft und/oder Sauerstoff und/oder Narkosegas gemischt werden, wobei die Gasdosierungseinheit (5) zumindest ein Ventil aufweist.

3. Beatmungsgerät (1) nach Anspruch 1, wobei der Pulsdämpfer" (6) eingerichtet ist, Impulswellen der Atemgasquelle (2) und/oder der Gasdosierungseinheit (5) zu dämpfen.

4. Beatmungsgerät (1) nach zumindest einem der vorhergehenden Ansprüche, wobei die pneumatische Hauptleitung (4) eine Durchflussrichtung (d) von der Gasdosierungseinheit (5) und dem Druckerzeuger (2) zum Patienteninterface aufweist, wobei die Gasdosierungseinheit (5) in Durchflussrichtung (d) vor dem Druckerzeuger (2) angeordnet ist und der Druckerzeuger (2) in Durchflussrichtung (d) vor dem Patienteninterface (9) oder der Druckerzeuger (2) in Durchflussrichtung (d) vor der Gasdosierungseinheit (5) angeordnet ist und die Gasdosierungseinheit (5) in Durchflussrichtung (d) vor dem Patienteninterface (9).

5. Beatmungsgerät (1) nach zumindest einem der vorhergehenden Ansprüche, wobei der Pulsdämpfer (6) pneumatisch zwischen der Gasdosierungseinheit (5) und der pneumatischen Hauptleitung (4) angeordnet ist und einen Anschluss (602) für die Gasdosierungseinheit 5 und einen Anschluss (603) für pneumatische Hauptleitung (4) aufweist.

6. Beatmungsgerät (1) nach zumindest einem der vorhergehenden Ansprüche, wobei der Pulsdämpfer (6) pneumatisch hinter der Gasdosierungseinheit (5) und/oder dem Druckerzeuger (2) in der pneumatischen Hauptleitung (4) angeordnet ist.

7. Beatmungsgerät (1) nach zumindest einem der vorhergehenden Ansprüche, wobei der Pulsdämpfer (6) so eingerichtet und ausgebildet ist, dass sich das Volumen (u₁) des pulsartig eingespeisten Atemgases in der Kammer (604) ausbreiten kann und in einen weniger pulsartigen oder in einen stetigen Fluss überführt wird.

8. Beatmungsgerät (1) nach zumindest einem der vorhergehenden Ansprüche, wobei der Pulsdämpfer (6) so eingerichtet und ausgebildet ist, dass sich das Volumen (u₁) des pulsartig eingespeisten Atemgases in der Kammer (604) ausbreiten kann und in einen weniger pulsartigen oder in einen stetigen Fluss überführt wird, wobei der entstehende, weniger pulsartige oder stetige Fluss angepasst an den Durchfluss in der pneumatischen Hauptleitung (4) ist und von 20 bis 100 L/min, bevorzugt von 30 bis 80 L/min, beträgt.

9. Beatmungsgerät (1) nach zumindest einem der vorhergehenden Ansprüche, wobei in der Kammer (604) eine Oberfläche gegeben ist, welche zumindest teilweise mit Dämmmaterial ausgekleidet ist.

10. Beatmungsgerät (1) nach zumindest einem der vorhergehenden Ansprüche, wobei die Dämpferstärke des Pulsdämpfers (6) durch Versetzen des zumindest einen Federelements (701, 702, 703) einstellbar ist.

11. Beatmungsgerät (1) nach zumindest einem der vorhergehenden Ansprüche, wobei das die Gehäuseteile (601, 801) über ein Scharnier (705) so miteinander verbunden sind, dass sich das Gehäuseteil (601) bei Expandieren des Pulsdämpfers (6) an einem Ende stärker anhebt als an dem gegenüberliegenden Ende.

12. Beatmungsgerät (1) nach zumindest einem der vorhergehenden Ansprüche, wobei die Dämpferstärke des Pulsdämpfers (6) über die Federstärke des zumindest einen Federelements (701, 702, 703) einstellbar ist.

## Claims

1. A ventilator (1) having a pressure generator (2), a gas-dosing unit (5) and a pneumatic main line (4) which guides respiratory gas to a patient interface, wherein
- the gas-dosing unit (5) is configured to guide external air, and/or oxygen and/or anesthetic gas as respiratory gas into the pneumatic main line (4), and
- the pressure generator (2) is configured to convey respiratory gas into the pneumatic main line to the patient interface (9),
wherein the gas-dosing unit (5) is configured to deliver determined amounts or volumes of respiratory gas into the pneumatic main line (4), wherein the ventilator has a pulsation dampener (6), wherein the pulsation dampener (6) is placed in a pulsation dampener housing, comprising an upper housing part (601) and a lower housing part (801), which hold the pulsation dampener (6) at least partially or in sections, wherein the pulsation dampener housing (601) has at least one spring element (701, 702, 703), **characterized in that** the pulsation dampener (6) is designed as an elastomer component with a tubular inlet from a first connection (602), which expands to a chamber (604), wherein the chamber (604) gradually transitions into a tubular outlet to a second connection (603).

2. The ventilator (1) according to claim 1, wherein the gas-dosing unit (5) is configured to provide respiratory gas by mixing air and/or oxygen and/or anesthetic gas, wherein the gas-dosing unit (5) has at least one valve.

3. The ventilator (1) according to claim 1, wherein the pulsation dampener (6) is configured to dampen pulse waves from the respiratory gas source (2) and/or from the gas-dosing unit (5).

4. The ventilator (1) according to at least one of the preceding claims, wherein the pneumatic main line (4) has a direction of flow (d) from the gas-dosing unit (5) and the pressure generator (2) to the patient interface, wherein the gas-dosing unit (5) is arranged upstream of the pressure generator (2) in the direction of flow (d) and the pressure generator (2) is arranged upstream of the patient interface (9) in the direction of flow (d) or the pressure generator (2) is arranged upstream of the gas-dosing unit (5) in the direction of flow (d) and the gas-dosing unit (5) is arranged upstream of the patient interface (9) in the direction of flow (d).

5. The ventilator (1) according to at least one of the preceding claims, wherein the pulsation dampener (6) is arranged pneumatically between the gas-dosing unit (5) and the pneumatic main line (4) and has a connection (602) for the gas-dosing unit (5) and a connection (603) for the pneumatic main line (4).

6. The ventilator (1) according to at least one of the preceding claims, wherein the pulsation dampener (6) is arranged pneumatically after the gas-dosing unit (5) and/or the pressure generator (2) in the pneumatic main line (4).

7. The ventilator (1) according to at least one of the preceding claims, wherein the pulsation dampener (6) is configured and designed such that the volume (u₁) of the respiratory gas in the chamber (604) that is fed in in pulses can expand and is converted into a less pulse-like or a constant flow.

8. The ventilator (1) according to at least one of the preceding claims, wherein the pulsation dampener (6) is configured and designed such that the volume (u₁) of the respiratory gas in the chamber (604) that is fed in in pulses can expand and is converted into a less pulse-like or a constant flow, wherein the resulting less pulse-like or constant flow is adjusted to the throughflow in the pneumatic main line (4) and is between 20 and 100 l/min, preferably between 30 and 80 l/min.

9. The ventilator (1) according to at least one of the preceding claims, wherein in the chamber (604) there is a surface which is at least partially lined with insulating material.

10. The ventilator (1) according to at least one of the preceding claims, wherein the damping strength of the pulsation dampener (6) can be adjusted by moving the at least one spring element (701, 702, 703).

11. The ventilator (1) according to at least one of the preceding claims, wherein the housing parts (601, 801) are connected to one another by means of a hinge (705), such that when the pulsation dampener (6) expands, one end of the housing part (601) is raised to a greater extent than the opposite end.

12. The ventilator (1) according to at least one of the preceding claims, wherein the damping strength of the pulsation dampener (6) can be adjusted by the spring strength of the at least one spring element (701, 702, 703).

## Revendications

1. Appareil respiratoire (1) comprenant un générateur de pression (2), une unité de dosage de gaz (5) et une conduite principale pneumatique (4), laquelle guide du gaz respiratoire vers une interface patient, dans lequel
- l'unité de dosage de gaz (5) est conçue pour guider de l'air ambiant et/ou de l'oxygène et/ou du gaz d'anesthésie comme gaz respiratoire dans la conduite principale pneumatique (4), et
- le générateur de pression (2) est conçu pour transporter du gaz respiratoire dans la conduite principale pneumatique vers l'interface patient (9),
dans lequel l'unité de dosage de gaz (5) est conçue pour délivrer des quantités ou des volumes prédéfinis de gaz respiratoire dans la conduite principale pneumatique (4), dans lequel l'appareil respiratoire présente un amortisseur d'impulsions (6), dans lequel l'amortisseur d'impulsions (6) est placé dans un boîtier d'amortisseur d'impulsions comportant une partie de boîtier supérieure (601) et une partie de boîtier inférieure (801), lesquelles supportent l'amortisseur d'impulsions (6) au moins partiellement ou par endroits, dans lequel le boîtier d'amortisseur d'impulsions (601) présente au moins un élément à ressort (701, 702, 703), **caractérisé en ce que** l'amortisseur d'impulsions (6) est formé comme un composant élastomère, avec une entrée tubulaire partant d'un premier raccord (602) s'élargissant en une chambre (604), dans lequel la chambre (604) se prolonge progressivement en une sortie tubulaire vers un deuxième raccord (603).

2. Appareil respiratoire (1) selon la revendication 1, dans lequel l'unité de dosage de gaz (5) est conçue pour fournir du gaz respiratoire par mélange d'air et/ou d'oxygène et/ou de gaz d'anesthésie, dans lequel l'unité de dosage de gaz (5) présente au moins une soupape.

3. Appareil respiratoire (1) selon la revendication 1, dans lequel l'amortisseur d'impulsions (6) est conçu pour amortir des ondes d'impulsion de la source de gaz respiratoire (2) et/ou de l'unité de dosage de gaz (5).

4. Appareil respiratoire (1) selon l'une au moins des revendications précédentes, dans lequel la conduite principale pneumatique (4) présente une direction d'écoulement (d) allant de l'unité de dosage de gaz (5) et du générateur de pression (2) vers l'interface patient, dans lequel l'unité de dosage de gaz (5) est disposée en amont du générateur de pression (2) dans la direction d'écoulement (d) et le générateur de pression (2) est disposé en amont de l'interface patient (9) dans la direction d'écoulement (d) ou le générateur de pression (2) est disposé en amont de l'unité de dosage de gaz (5) dans la direction d'écoulement (d) et l'unité de dosage de gaz (5) est disposée en amont de l'interface patient (9) dans la direction d'écoulement (d).

5. Appareil respiratoire (1) selon l'une au moins des revendications précédentes, dans lequel l'amortisseur d'impulsions (6) est disposé pneumatiquement entre l'unité de dosage de gaz (5) et la conduite principale pneumatique (4) et présente un raccord (602) pour l'unité de dosage de gaz (5) et un raccord (603) pour la conduite principale pneumatique (4).

6. Appareil respiratoire (1) selon l'une au moins des revendications précédentes, dans lequel l'amortisseur d'impulsions (6) est disposé pneumatiquement en aval de l'unité de dosage de gaz (5) et/ou du générateur de pression (2) dans la conduite principale pneumatique (4).

7. Appareil respiratoire (1) selon l'une au moins des revendications précédentes, dans lequel l'amortisseur d'impulsions (6) est conçu et formé de telle façon que le volume (u₁) du gaz respiratoire introduit par impulsions dans la chambre (604) peut se dilater et est transféré dans un flux moins pulsé ou constant.

8. Appareil respiratoire (1) selon l'une au moins des revendications précédentes, dans lequel l'amortisseur d'impulsions (6) est conçu et formé de telle façon que le volume (u₁) du gaz respiratoire introduit par impulsions dans la chambre (604) peut se dilater et est transféré dans un flux moins pulsé ou constant, dans lequel le flux moins pulsé ou constant résultant est adapté à l'écoulement dans la conduite principale pneumatique (4) et compris entre 20 et 100 L/min, de préférence entre 30 et 80 L/min.

9. Appareil respiratoire (1) selon l'une au moins des revendications précédentes, dans lequel une surface au moins partiellement revêtue de matériau isolant est prévue dans la chambre (604).

10. Appareil respiratoire (1) selon l'une au moins des revendications précédentes, dans lequel la puissance d'amortissement de l'amortisseur d'impulsions (6) peut être réglée par déplacement de l'au moins un élément à ressort (701, 702, 703).

11. Appareil respiratoire (1) selon l'une au moins des revendications précédentes, dans lequel les parties de boîtier (601, 801) sont reliées entre elles de telle façon par le biais d'une charnière (705), que la partie de boîtier (601) s'élève davantage à une extrémité qu'à l'extrémité opposée lors de l'expansion de l'amortisseur d'impulsions (6).

12. Appareil respiratoire (1) selon l'une au moins des revendications précédentes, dans lequel la puissance d'amortissement de l'amortisseur d'impulsions (6) peut être réglée par la force de ressort de l'au moins un élément à ressort (701, 702, 703).
